# EUROPEAN PATENT APPLICATION

(11) **EP 4 098 254 A1**
(43) Date of publication of application: **07.12.2022**
(21) Application number: 21305752.4
(22) Date of filing: 04.06.2021
(51) Int. Cl.: A61K 31/05, A61P 29/02, A61K 9/00

(54) **CANNABIDIOL FOR USE IN THE TREATMENT OF PAIN RESULTING FROM AN INDOLEAMINE 2,3-DIOXYGENASE-1 (IDO1) RELATED DISEASE**

(71) Applicant: ASSISTANCE PUBLIQUE, HOPITAUX DE PARIS, 75004 Paris (FR)
(72) Inventor: GRECO, Céline, 75012 PARIS (FR); KHOUKH, Karim, 92100 BOULOGNE-BILLANCOURT (FR); BRUNO, Fabien, 75006 PARIS (FR)
(74) Representative: Gevers & Orès

(57) **Abstract**

The present invention relates to cannabidiol (CBD), prodrugs thereof and pharmaceutically acceptable salts thereof, or a composition comprising such a compound, for use in the treatment or prevention of pain caused by an indoleamine 2,3-dioxygenase-1 (IDOl) related disease.

## Description

The present invention relates to cannabidiol (CBD), prodrugs thereof and pharmaceutically acceptable salts thereof, or a composition comprising such a compound, for use in the treatment or prevention of pain caused by an indoleamine 2,3-dioxygenase-1 (IDO1) related disease.

IDO1 is a member of a unique class of dioxygenases that catalyze the oxidative catabolism of L-tryptophan, the least-abundant essential amino acid, along the kynurenine pathway. In normal conditions, this enzyme participates in biological functions by breaking down tryptophan or by regulating T-cell populations. However, an elevated IDO activity is also associated to some cancers and various inflammatory or metabolic diseases, such as mastocytosis and Mast Cell Activation Syndrome (MCAS), Crohn's disease, myeloma or rheumatoid arthritis.

IDO1 related diseases are pathologies for which analgesic solutions are poor or not very effective. Moreover, the actual treatments, mainly based on strong opioids, antidepressants or anti-epileptic drugs, are often associated with side effects.

There thus remains a genuine need for new treatments that would be more efficient and would have no or a less side effects compared with actual solutions.

The present invention is believed to meet such need by providing a composition for treating or preventing pain caused by an IDOl related disease.

Cannabidiol (CBD) is the second most prevalent of the active ingredients of cannabis. While CBD is a component of medical marijuana, it is derived directly from the hemp plant, which is a cousin of the marijuana plant. CBD, unlike Tetrahydrocannabinol (Δ9-THC), is a non psycho-active compound. It is commonly used to address anxiety. Various studies also suggest that CBD has a positive effect in managing and treating chronic pain, posttraumatic stress disorder, depression, diabetes or seizures. So far, no toxic dose for CBD is known and there is no record of a death or serious injury from CBD.

In a surprising manner, the Inventors have demonstrated that CBD can be used in the treatment or prevention of pain caused by an IDOl related disease, with no side effects compared with actual treatments.

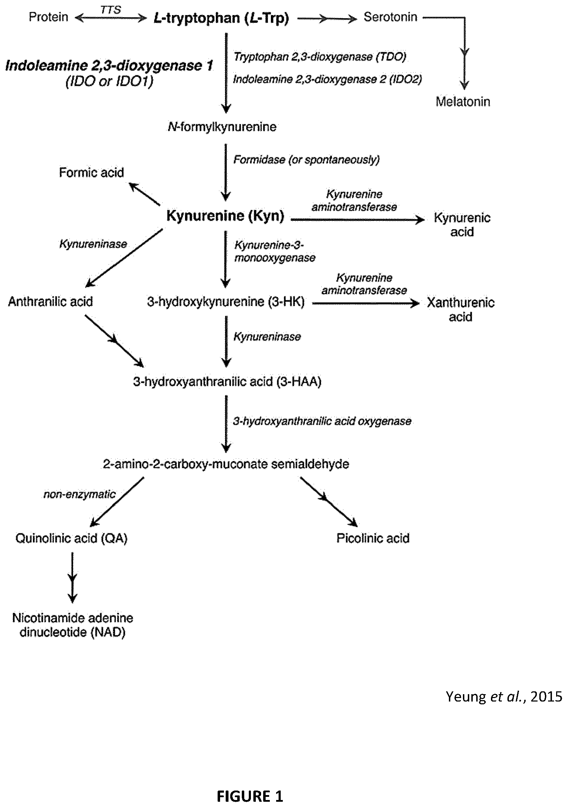

Without intending to be bound to any theory, it is proposed by the Inventors that CBD is efficient to reduce pain caused by an IDOl related disease since it has a suppressive effect on tryptophan degradation mediated by IDO1. Indeed, the high activity of IDOl leads to an increase in the production of quinolinic acid (Figure 1), which is an agonist of N-methyl-D-aspartate (NMDA) receptors, i.e. the fundamental receptors involved in pain. Thus, in pathologies involving an increased activity of IDOl, the NMDA receptors are over-activated and patients suffer pain.

Therefore, the present invention concerns a compound selected from cannabidiol (CBD), prodrugs thereof and pharmaceutically acceptable salts thereof, for use in the treatment or prevention of pain caused by an indoleamine 2,3-dioxygenase-1 (IDOl) related disease.

The present invention further relates to a composition comprising a compound according to the invention, as active ingredient and at least one pharmaceutically acceptable excipient, for use in the treatment or prevention of pain caused by an IDOl related disease.

The present invention further relates to a method for treating and/or preventing pain caused by an IDOl related disease by means of administration, to a patient in need thereof, of an effective amount of a compound or a composition according to the invention.

The present invention further relates to the use of a compound or composition comprising a compound according to the invention for the manufacture of a medication for treating and/or preventing pain caused by an IDOl related disease.

"Cannabidiol" (or "CBD") as used herein refers to the molecule of formula (I), enantiomers, racemic mixtures, polymorphs, salts, solvates, esters or hydrates thereof.

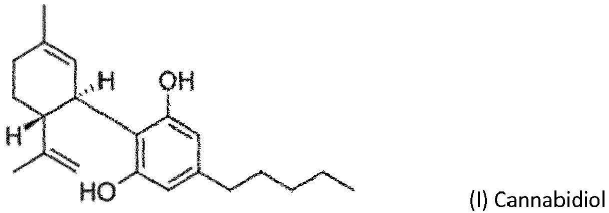

In the present invention, "prodrug" designates a compound that, after administration, is metabolized (i.e. converted within the body) into a pharmacologically active drug.

In the present invention, "pharmaceutically acceptable" is intended to mean that which is useful in the preparation of a pharmaceutical composition, generally safe, nontoxic and neither biologically nor otherwise undesirable, and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

By "pharmaceutically acceptable excipient" is meant, according to the invention, an excipient that is compatible with the other ingredients of the composition and that produces no adverse effect, allergic reaction or other undesirable reaction when it is administered to a human or an animal.

In the present invention, the term "indoleamine 2,3-dioxygenase-1 related disease" (or "IDO1 related disease") designates a disease that is associated with an elevated expression or an elevated activity of the enzyme IDO1. Non-limitative examples of IDO related diseases are given in Yeung et al., 2015 (Clinical Science 129, 601-672).

Preferably, the IDO1 related disease is a disease chosen from the group comprising: mastocytosis, in particular indolent systemic mastocytosis (ISM), mast cell activation syndrome (MCAS), Crohn's disease, myeloma and rheumatoid arthritis.

"Mastocytosis" is a rare disorder characterized by abnormal accumulations of mast cells in the skin, bone marrow, and internal organs (liver, spleen, gastrointestinal tract and lymph nodes). Cases beginning during adulthood tend to be chronic and involve the bone marrow in addition to the skin, whereas, during childhood, the condition is often marked by skin manifestations with no internal organ involvement and can often resolve during puberty. In most adult patients, mastocytosis tends to be persistent, and may progress into a more advanced category in a minority of patients.

"Systemic mastocytosis" is the main form of mastocytosis observed in adults whereas it is rarer in children. Systemic disease is defined by demonstration of pathologic accumulation of mast cells in a tissue other than skin (most commonly bone marrow). Hypertryptasemia, i.e. elevated basal serum tryptase levels, usually occurs in systemic mastocytosis

"Indolent systemic mastocytosis" (or "ISM") is a form of systemic mastocytosis generally associated with low mast cell burden, and presence of mediator-related symptoms. Most patients also have maculopapular skin lesions. Some patients may present an enlarged liver or spleen and the gastrointestinal tract may also be affected. Symptoms include muscle and joint pain, skin reactions such as itching and flushing, and gut symptoms such as being sick and diarrhea.

"Mast cell activation syndrome" (or "MCAS") is a condition in which the patient experiences repeated episodes of the symptoms of anaphylaxis. It is caused by the inappropriate release of mast cell mediators including histamine, interleukins, prostaglandins, cytokines, chemokines, and heparin. Symptoms include episodes of abdominal pain, cramping, diarrhea, flushing, itching, wheezing, coughing, lightheadedness and rapid pulse and low blood pressure.

"Crohn's disease" is a type of inflammatory bowel disease (IBD) that may affect any segment of the gastrointestinal tract from the mouth to the anus. Symptoms often include abdominal pain, diarrhea (which may be bloody if inflammation is severe), fever, abdominal distension, and weight loss.

"Myeloma" (or "multiple myeloma") is a cancer that forms in plasma cells. In myeloma, cancerous plasma cells accumulate in the bone marrow and crowd out healthy blood cells. Rather than produce helpful antibodies, the cancer cells produce abnormal proteins that can cause complications. Symptoms include bone pain, especially in spine or chest, nausea, constipation, loss of appetite, mental fogginess or confusion, fatigue, frequent infections, weight loss, weakness or numbness in legs and excessive thirst.

"Rheumatoid arthritis" is a long-term autoimmune disorder that primarily affects joints. Symptoms include pain or aching in more than one joint, stiffness in more than one joint, tenderness and swelling in more than one joint, weight loss, fever, fatigue and weakness.

By "treatment" is meant, according to the present invention, the inhibition of the development of, more particularly the regression of, preferably the disappearance of the pain caused by an IDO1 related disease.

By "prevention" is meant, according to the present invention, to prevent or delay the appearance of the pain caused by an IDO1 related disease.

In particular, the composition of the invention is appropriate for treating or preventing musculoskeletal, abdominal or neuropathic pain caused by mastocytosis or MCAS.

In particular, the composition of the invention is appropriate for treating or preventing abdominal pain caused by Crohn's disease.

In particular, the composition of the invention is appropriate for treating or preventing musculoskeletal pain caused by a myeloma.

In particular, the composition of the invention is appropriate for treating or preventing abdominal pain, abdominal pain or neuropathic pain caused by Rheumatoid arthritis.

The treatment or prevention according to the invention applies to animals and humans, adults or children.

The composition of the invention can be in any form allowing an administration by enteral, parenteral or topical routes.

The composition according to the invention may be in any form, such as a solution, a syrup, a powder, a pill, a capsule, a suppository, a cream, a gel, an ointment, a solution, a lotion, a spray, an aerosol spray, an aerosol foam or a patch.

In an embodiment, the composition is in the form of a pill or a capsule for an enteral administration, preferably an oral administration.

In an embodiment, the composition is in the form of a lotion, a cream or a gel for a topical administration by application on the skin surface or the mucous membranes.

In an embodiment, the composition of the invention can be topically applied to the skin by an applicator, such as a roll-on, a stick, an impregnated wipe or an impregnated glove.

In an embodiment, the composition of the invention can also be dispensed from a pump pack or from an aerosol container.

In an embodiment, the composition of the invention for topical administration comprises a compound according to the invention in a concentration from 0.1 to 30%, in particular from 0.5% to 5%, more particularly from 1% to 2%, by weight relative to the weight of the final composition.

In an embodiment, the composition of the invention for topical administration comprises a compound according to the invention in a concentration of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the final composition.

In an embodiment, the composition of the invention is a lotion, a cream or a gel comprising a compound according to the invention in a concentration from 0.5% to 5%.

In an embodiment, the composition of the invention is a lotion, a cream or a gel comprising a compound according to the invention in a concentration of 0.5%.

In an embodiment, the composition of the invention is a lotion, a cream or a gel comprising a compound according to the invention in a concentration of 1%.

In an embodiment, the composition of the invention is a lotion, a cream or a gel comprising a compound according to the invention in a concentration of 2%.

In an embodiment, the composition of the invention is a lotion, a cream or a gel comprising a compound according to the invention in a concentration of 5%.

In an embodiment, the composition of the invention for oral administration comprises a compound according to the invention in a unit dose amount from 1 to 3 000 mg, in particular from 50 mg to 2 000 mg, more particularly from 150 mg to 1 500 mg.

In an embodiment, the composition of the invention for oral administration comprises a compound according to the invention in a unit dose amount from 100 to 200 mg.

In an embodiment, the composition of the invention for oral administration comprises a compound according to the invention in a unit dose amount of 1 mg, 2 mg, 3 mg, 4 mg, 5 mg, 6 mg, 7 mg, 8 mg, 9 mg, 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1 000 mg, 1 100 mg, 1 200 mg, 1 300 mg, 1 400 mg, 1 500 mg, 1 600 mg, 1 700 mg, 1 800 mg, 1 900 mg, 2 000 mg, 2 100 mg, 2 200 mg, 2 300 mg, 2 400 mg, 2 500 mg, 2 600 mg, 2 700 mg, 2 800 mg, 2 900 mg or 3 000 mg.

In a preferred embodiment, the composition of the invention is a capsule or a pill comprising a compound according to the invention in an amount of 10 mg.

In a preferred embodiment, the composition of the invention is a capsule or a pill comprising a compound according to the invention in an amount of 30 mg.

In a preferred embodiment, the composition of the invention is a capsule or a pill comprising a compound according to the invention in an amount of 50 mg.

In a preferred embodiment, the composition of the invention is a capsule or a pill comprising a compound according to the invention in an amount of 100 mg.

In a preferred embodiment, the composition of the invention is a capsule or a pill comprising a compound according to the invention in an amount of 200 mg.

In a preferred embodiment, the composition of the invention is a capsule or a pill comprising a compound according to the invention in an amount of 300 mg.

In a preferred embodiment, the composition of the invention is a capsule or a pill comprising a compound according to the invention in an amount of 400 mg.

In a preferred embodiment, the composition of the invention is a capsule or a pill comprising a compound according to the invention in an amount of 500 mg.

In a preferred embodiment, the composition of the invention is a capsule or a pill comprising a compound according to the invention in an amount of 1 000 mg.

In a preferred embodiment, the composition of the invention is a capsule or a pill comprising a compound according to the invention in an amount of 1 500 mg.

In a preferred embodiment, the composition of the invention is a capsule or a pill comprising a compound according to the invention in an amount of 2 000 mg.

In a preferred embodiment, the composition of the invention is a capsule or a pill comprising a compound according to the invention in an amount of 2 500 mg.

In a preferred embodiment, the composition of the invention is a capsule or a pill comprising a compound according to the invention in an amount of 3 000 mg.

In an embodiment, the composition of the invention is formulated into unit dose form.

In an embodiment, the compound or composition of the invention is administered at a regimen of a dose from 1 to 3 000 mg per day, in particular a dose from 50 mg to 2 000 mg per day, more particularly a dose from 150 mg to 1 500 mg per day.

In an embodiment, the compound or composition of the invention is administered at a regimen of a dose from 150 to 1500 mg per day, preferably by oral administration.

In an embodiment, the compound or composition of the invention is administered at once at a dose from 150 to 1 500 mg per day, preferably by oral administration.

The compound or composition according to the invention will be administered one or more times per day, the duration being variable according to the pain intensity and easily adjustable by the person skilled in the art or the practitioner.

In an embodiment, the compound or composition of the invention is administered once daily, twice daily, three times daily, four times daily, once every second day, three times weekly, twice weekly or once weekly.

In an embodiment, the compound or composition of the invention is administered for at least one month, preferably at least two months, more preferably at least three months.

In an embodiment, the compound or composition of the invention is administered before a pain episode.

In an embodiment, the compound or composition of the invention is administered during a pain episode.

In an embodiment, the invention relates to a composition comprising a compound according to the invention, as active ingredient and at least one pharmaceutically acceptable excipient, for treating or preventing pain caused by a disease chosen from the group comprising: mastocytosis, in particular indolent systemic mastocytosis (ISM), mast cell activation syndrome (MCAS), Crohn's disease, myeloma, rheumatoid arthritis, said composition being orally administered once per a day in the form of capsule or a pill comprising from 1 to 3 000 mg, preferably from 150 to 1 500 mg of said compound.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Kynurenine pathway of L-Trp metabolism.

### EXAMPLES

### Example 1 - Treatment of 15 patients suffering from ISM, hypertryptasemia or MCAS

Fifteen patients suffering from indolent systemic mastocytosis (ISM) or mast cell activation syndrome (MCAS) or having hypertryptasemia with no positive result from classic treatments were treated with a daily dose from 15 to 400 mg of cannabidiol (CBD) by capsule.

The pain was measured using numeric rating scale (NRS) pain score, between 0 (no pain) and 10 (worst pain imaginable).

At day 0 (D0), the average pain score for the 15 patients was about 7.2.

After one month of treatment (M1), a reduction of pain of about 2 points was observed (average pain score of 5.1). A relief of pain was observed for 13 of 15 patients.

After three months of treatment (M3), a reduction of pain of about 3 points was observed (average pain score of 3.8). A relief of pain was observed for 14 of 15 patients.

The results are shown in Table 1.

**Table 1. Details and evolution of pain for 15 patients treated with CBD.**

| **Patient** | **Age** | **Pathology** | **Minor WHO Criteria** | **NRS D0** | **NRS M1** | **NRS M3** | **CBD posology (mg/day)** | **pain treatment withdrawal** |
|---|---|---|---|---|---|---|---|---|
| 01-009 Female | 37 | ISM | cKIT D816V + in cavum biopsy | 6 | 3 | 2 | 40 | stop dantrolene and acetaminophen |
| 01-011 Female | 37 | ISM | mast cells in digestive tract expressing CD25 with CD2 | 9 | 2 | 5 | 60 | acetaminophen if pain |
| 01-012 Male | 59 | ISM | cKIT D816V + in bone marrow | 8 | 5 | 1 | 15 | |
| 01-018 Male | 54 | Hypertryptasemia | tryptase 15ng/ml | 6 | 6 | 6 | 70 | stop stelara |
| 01-021 Male | 56 | ISM | mast cells in digestive tract expressing CD25 with CD2 | 8 | 3 | 6,5 | 300 | stop NSAID |
| 01-022 Female | 16 | Hypertryptasemia | tryptase 22ng/ml | 8 | 6 | 2 | 150 | stop nefopam |
| 01-025 Female | 53 | ISM | tryptase 36ng/ml | 8 | 5 | 7 | 180 | stop memantine, ketamine, dantrolene, thiocolcchicoside |
| 01-027 Male | 46 | MCAS | | 7 | 8 | 4 | 300 | stop opioids |
| 01-028 Female | 60 | ISM | tryptase 154ng/ml | 8,5 | 8 | 2 | 150 | stop methadone, duloxetine, pregabalin |
| 01-032 Female | 34 | ISM | mast cells in digestive tract expressing CD25 with CD2 | 5 | 5 | 3 | 200 | stop methadone |
| 01-029 Female | 30 | MCAS | | 5 | 4 | 4 | 80 | |
| 01-035 Female | 42 | MCAS | | 8 | 7 | 5 | 90 | stop ketamine |
| 01-004 Female | 33 | ISM | cKIT D816V + in bone marrow, >25% of the mast cells in the infiltrate are spindle-shaped | 7 | 3,5 | 2,5 | 60 | stop duloxetine and pregabalin |
| 01-020 Female | 66 | ISM | cKIT D816V + in bone marrow, tryptase 33ng/ml | 7 | 6 | 3 | 60 | stop amitriptyline |
| 01-049 Female | 47 | ISM | cKIT D816V + in bone marrow | 7 | 5 | 4 | 400 | stop methadone |

## Claims

1. A compound selected from cannabidiol (CBD), prodrugs thereof and pharmaceutically acceptable salts thereof, for use in the treatment or prevention of pain caused by an indoleamine 2,3-dioxygenase-1 (IDO1) related disease.

2. A composition comprising a compound selected from cannabidiol (CBD), prodrugs thereof and pharmaceutically acceptable salts thereof, as active ingredient and at least one pharmaceutically acceptable excipient, for use in the treatment or prevention of pain caused by an IDO1 related disease.

3. The compound for use according to claim 1 or the composition for use according to claim 2, wherein said IDO1 related disease is chosen from the group comprising mastocytosis, in particular indolent systemic mastocytosis (ISM), mast cell activation syndrome (MCAS), Crohn's disease, myeloma and rheumatoid arthritis.

4. The compound for use according to claim 1 or 3 or the composition for use according to claim 2 or 3, wherein the compound is administered at a regimen of a dose from 1 to 3 000 mg per day, in particular a dose from 50 mg to 2 000 mg per day, more particularly a dose from 150 mg to 1 500 mg per day.

5. The compound for use according to any one of claims 1 or 3-4 or the composition for use according to any one of claims 2-4, wherein the compound is administered at once at a dose from 150 to 1 500 mg per day, preferably by oral administration.

6. The composition for use according to any one of claims 2-5, wherein the composition is in a form allowing an administration by enteral, parenteral or topical routes.

7. The composition for use according to any one of claims 2-6, wherein the composition is in the form of a pill or a capsule for an enteral administration, preferably an oral administration.

8. The composition for use according to any one of claims 2-6, wherein the composition is in the form of a lotion, a cream or a gel for a topical administration by application on the skin surface or the mucous membranes.
